# EUROPEAN PATENT SPECIFICATION

(11) **EP 3 166 655 B1**
(45) Date of publication and mention of the grant of the patent: **03.07.2019**
(21) Application number: 15753188.0
(22) Date of filing: 07.07.2015
(51) Int. Cl.: A61M 1/00

(54) **PRESSURE REDUCER FOR SURGICAL ASPIRATION SYSTEMS**
DRUCKMINDERER FÜR CHIRURGISCHE ASPIRATIONSSYSTEME
DISPOSITIF DE RÉDUCTION DE PRESSION POUR DES SYSTÈMES D'ASPIRATION CHIRURGICALE

(30) Priority: 07.07.2014 IT MO20140191
(43) Date of publication of application: 17.05.2017
(73) Proprietor: Promev S.r.l., 23900 Lecco (LC) (IT)
(72) Inventor: DALLOLIO, Villiam, I-23900 Lecco (IT)
(74) Representative: Brunacci, Marco
(86) International application number: PCT/IB2015/055124
(87) International publication number: WO 2016/005894

(56) References cited:
- DE-A1- 3 015 399
- US-A- 3 834 388
- US-A- 5 013 300
- US-A- 5 730 727
- US-A- 5 890 516
- US-A1- 2003 216 690
- US-A1- 2010 282 253

## Description

### Technical Field

The present invention relates to a pressure reducer for surgical aspiration systems, usable in all the fields of surgery and in particular in the field of neurosurgery.

### Background Art

In every operating theater there is an aspirating instrument, connected to an aspirating machine, with which the surgeon can remove the biological fluids, in particular blood, from the operating area.

The aspirating pressure must be regulated according to the conditions in which the surgeon has to operate, the quantity of liquid to be aspirated and the areas of the body where operations are performed.

In the case of neurosurgical operations, the areas in which operations are performed are very delicate, so it is necessary for the aspirating pressure to be regulated in a precise and safe way.

A first type of pressure reducer is known used in the operating theater.

These first known reducers are placed on the aspirating instrument used by the surgeon and are composed of rigid tubular elements, usually made of metal, which have a hole on the outer surface that connects the internal part of the tubular element to the outside.

The surgeon regulates the pressure manually by closing the hole with a finger and in a gradual manner depending on the pressure variation to be achieved.

A second type of known reducer consists of a handmade tubular article having three holes similar to that described for the first known type.

This article has two ends connected, by means of two generic tubes, to the aspirating machine and to the aspirating instrument used by the surgeon.

The operating theater staff, supervised by the surgeon, is charged with closing or opening the holes according to the regulation to be obtained.

These known reducers have a number of drawbacks.

In the first type of reducer it is the surgeon him/herself who must make the pressure reduction. This, besides not being a very precise and approximate operation, involves risks in terms of safety.

The surgeon, in fact, to regulate the closing of the hole, could make movements with the aspirating instrument, movements that could prove very dangerous in some types of operations in which the utmost attention is required (operations on aneurysms, brain tumors, etc.).

The second type of reducer has a drawback linked to the possible blockage of the holes.

During the aspirating phase, in fact, some residues of aspirated liquids may be retained in the holes and, on hardening, cause their occlusion.

This occlusion adversely affects the ability to modulate the aspirating pressure. Another drawback is linked to the fact that in this case too pressure regulation is inconvenient and inaccurate.

Others devices are known from US5890516, US3834388, US2003/216690, US5730727, US2010/282253, DE3015399, US5013300.

### Description of the Invention

The main aim of the present invention is to provide a reducer which allows to regulate the aspirating pressure in a progressive and safe way.

Another aim of the present invention is to provide a reducer which allows a pressure regulation with ease of execution and control by the surgeon.

Another object of the present invention is to provide a reducer which allows to overcome the mentioned drawbacks of the prior art within the ambit of a simple, rational, easy, effective to use as well as affordable solution.

The objects stated above are achieved by the present pressure reducer having the characteristics of claim 1.

### Brief Description of the Drawings

Other characteristics and advantages of the present invention will become better evident from the description of a preferred, but not exclusive embodiment of a pressure reducer, illustrated by way of an indicative, but non-limiting example in the accompanying drawings, in which:
Figure 1 is an axonometric view of the reducer according to the invention, with all the closing elements in the opened position;
Figure 2 is an axonometric view of the reducer according to the invention, with two closing elements in the closed position;
Figure 3 is a sectional view perpendicular to the tubular body of the reducer according to the invention, at a closing element in the closed position and according to the section plane III-III of Figure 2.

### Embodiments of the Invention

With particular reference to such illustrations, reference number 1 globally indicates a pressure reducer for surgical aspiration systems.

The reducer 1 comprises at least a substantially elongated tubular body 2.

The tubular body 2 has at least a first end 3 connectable to at least a surgical aspirating unit and at least a second end 4 connectable to at least an end aspirating element.

By aspirating unit is meant, in particular, the machine that controls aspirating. Each operating theater is equipped with aspirating systems linked to one or more machines of this type.

The end aspirating element is usually the end instrument conventionally used by the surgeon for aspiration in correspondence of the operated parts.

The reducer 1 is characterized by the fact that the tubular body 2 comprises a plurality of holes 5 for regulating the aspirating pressure.

The holes 5 allow connecting the internal part of the cylindrical body to the outside to allow the air to come out and, therefore, a reduction in aspirating pressure. Furthermore, the reducer 1 comprises a plurality of closing elements 6 movable between a closed position of the regulating holes 5 and an opened position of the regulating holes 5. In particular, the closing elements 6 substantially are caps which in the closed position close the holes 5. The closing elements 6 also comprise at least a gripping portion 7.

In particular, the gripping portion 7 comprises a surface shaping 8 able to facilitate the grip.

In the present embodiment, the gripping portion 7 has a surface shaping 8 which defines two diametrically opposite recesses.

Alternative embodiments cannot be ruled out wherein such shaping 8 is made up e.g. of knurls or extrusions obtained directly on the surface of the gripping portion 7.

Besides the gripping portion 7, the closing elements 6 comprise at least a protruding portion 9 insertable snugly into the regulating holes 5.

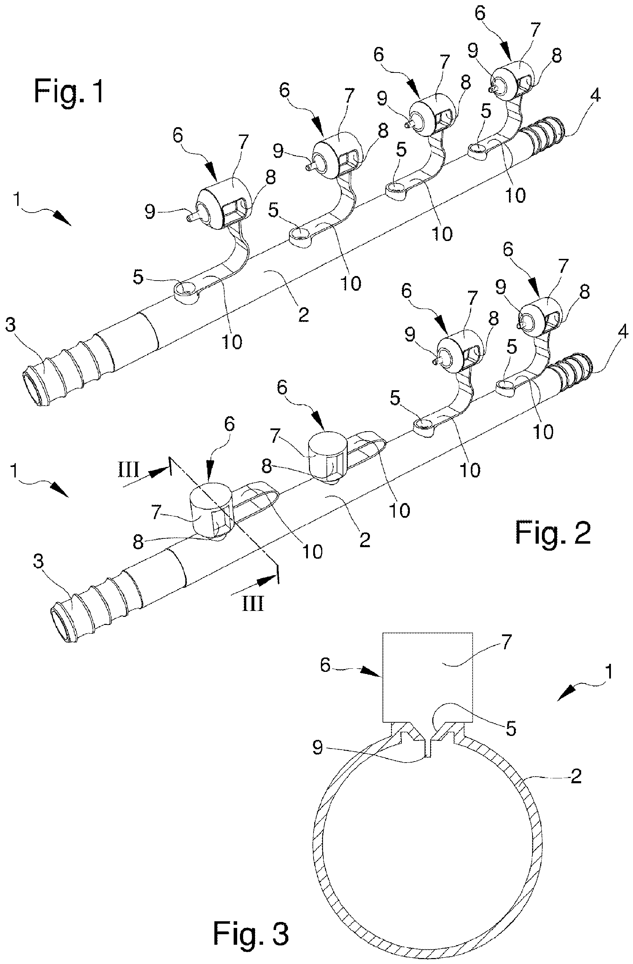

In the present embodiment, the protruding portion 9 has a substantially cylindrical shape and, with reference to the closed position shown in figure 3, is arranged below the gripping portion 7.

In particular, in the closed position, the protruding portion 9 of the closing elements 6 are inserted and interlocked inside the regulating holes 5.

Conveniently, as shown in figure 3, in the closed position, the protruding portion 9 extends at least partially beyond the hole 5, inside the tubular body 2. This way, any occlusions, incrustations and clots can be easily removed, by simply closing the hole 5 with the closing element 6.

With reference to a preferred embodiment, the protruding portion 9 extends inside the tubular body 2 for about 1 mm.

According to the invention, the reducer 1 comprises at least a flexible element 10 having a first end associated with the closing element 6 and a second end associated with the tubular body 2.

As shown in the attached illustrations, the flexible element 10 binds the closing element 6 to the tubular body 2.

According to the invention, the reducer 1 comprises a plurality of regulating holes 5 and a plurality of respective closing elements 6.

In particular, in the embodiment shown in figures 1 and 2, there are four holes 5 with the respective closing elements 6.

Still referring to the present embodiment, the holes 5 are machined to facilitate their closure.

In particular, as shown in figure 3, the holes 5 have a funnel inlet able to couple with a corresponding truncated-cone shaped portion of the protruding portion 9, so as to facilitate the entry of the protruding portion itself as far as the closed position.

The holes 5 are also aligned along the longitudinal axis of the tubular body 2 and spaced from one another.

In the present embodiment the holes 5 are equally spaced, but different arrangements cannot be ruled out.

In this way, to the opening of each hole 5 corresponds a pressure reduction of about 25% compared to the initial aspirating pressure.

With reference to figures 1 and 2, the first end 3 and the second end 4 of the reducer 1 comprise at least a coupling portion able to facilitate the connections with the aspirating machine and with the end aspirating element.

More particularly, these ends 3, 4 are suitably machined to allow easy insertion in the aspiration connecting tubes available in operating theaters and ensure their seal.

The operation of the present invention is as follows.

The reducer 1 is connected to the aspirating element and to the surgical aspirating unit by inserting the ends 3, 4 in the respective connecting tubes present in the operating theater.

Initially, the closing elements 6 are all in the closed position.

This way the surgeon begins to operate under conditions of maximum aspirating pressure.

Following the instructions of the surgeon, the staff can progressively open one or more holes 5 by simply lifting the closing element 6 by means of the gripping portion 7.

To each opened hole 5 corresponds a pressure reduction.

With reference to the embodiment shown in the Figures 1 and 2, to each opened hole 5 corresponds a pressure reduction of about 25%.

At the beginning of the operation, where aspiration usually must be greatest, the surgeon asks for all the holes 5 to be closed.

For each pressure reduction request, the holes 5 are gradually opened until reaching the minimum pressure which is obtained when all the holes 5 are open. Similarly, the staff can close the holes 5 by simply inserting the closing elements 6 in the holes 5 themselves.

This way, besides increasing the aspirating pressure, the hole 5 is cleaned due to the action of the protruding portion 9 of the closing element 6.

It has in practice been ascertained how the described invention achieves the intended objects and, in particular, the fact is underlined that this reducer allows to regulate the modular pressure in a progressive and safe way.

The reducer also allows easy regulation and easy to control by the surgeon.

The surgeon, in fact, because he/she knows the corresponding pressure reduction for each opened hole, is able to vocally command an auxiliary operator who can operate the reducer, for example, remotely.

Furthermore, the particular protruding portions allow, whenever the holes are closed, the self-cleaning of the reducer with removal of incrustations and occlusions present in the holes themselves, ensuring that the reducer operates properly for a long period of time.

All this in the ambit of a simple, rational, easy and effective to use as well as affordable solution.

## Claims

1. Pressure reducer (1) for surgical aspiration systems, comprising at least a substantially elongated tubular body (2) having at least a first end (3) connectable to at least a surgical aspirating unit and at least a second end (4) connectable to at least an end aspirating element, said tubular body (2) comprises at least a hole (5) for regulating the aspirating pressure, and said reducer (1) comprises at least a closing element (6) movable between a closed position of said regulating hole (5) and an opened position of said regulating hole (5), **characterized by** the fact that it comprises a plurality of regulating holes (5) and a plurality of respective closing elements (6).

2. Reducer (1) according to claim 1, **characterized by** the fact that said closing element (6) comprises at least a gripping portion (7).

3. Reducer (1) according to claim 2, **characterized by** the fact that said gripping portion (7) comprises a surface shaping (8) able to facilitate the grip.

4. Reducer (1) according to one or more of the preceding claims, **characterized by** the fact that said closing element (6) comprises at least a protruding portion (9) insertable snugly into said regulating hole (5).

5. Reducer (1) according to claim 4, **characterized by** the fact that, in said closed position, said protruding portion (9) of the closing element is inserted and interlocked inside said regulating hole (5).

6. Reducer (1) according to one or more of the claims 4 and 5, **characterized by** the fact that said protruding portion (9), in said closed position, extends at least partially beyond said hole (5), inside said tubular body (2).

7. Reducer (1) according to one or more of the preceding claims, **characterized by** the fact that it comprises at least a flexible element (10) having a first end associated with said closing element (6) and a second end associated with said tubular body (2).

8. Reducer (1) according to one or more of the preceding claims, **characterized by** the fact that said holes (5) are aligned along the longitudinal axis of said tubular body (2) and are substantially spaced from one another.

9. Reducer (1) according to one or more of the preceding claims, **characterized by** the fact that at least one of said first end (3) and said second end (4) comprises at least a coupling portion able to facilitate the connections with said aspirating machine and/or with said end element.

## Patentansprüche

1. Druckminderer (1) für chirurgische Aspirationssysteme, umfassend zumindest einen im Wesentlichen länglichen röhrenförmigen Körper (2) mit zumindest einem ersten Ende (3), das mit zumindest einer chirurgischen Aspirationseinheit verbindbar ist, und zumindest einem zweiten Ende (4), das mit zumindest einem Endaspirationselement verbindbar ist, wobei der röhrenförmige Körper (2) zumindest eine Öffnung (5) zur Regulierung des Aspirationsdrucks umfasst, und wobei der Druckminderer (1) zumindest ein Verschlusselement (6) umfasst, das zwischen einer geschlossenen Position der Regulierungsöffnung (5) und einer geöffneten Position der Regulierungsöffnung (5) bewegbar ist, **dadurch gekennzeichnet, dass** er eine Mehrzahl von Regulierungsöffnungen (5) und eine Mehrzahl von jeweiligen Verschlusselementen (6) umfasst.

2. Minderer (1) nach Anspruch 1, **dadurch gekennzeichnet, dass** das Verschlusselement (6) zumindest einen Griffabschnitt (7) umfasst.

3. Minderer (1) nach Anspruch 2, **dadurch gekennzeichnet, dass** der Griffabschnitt (7) eine Oberflächenformung (8) umfasst, die geeignet ist, den Griff zu erleichtern.

4. Minderer (1) nach einem oder mehreren der vorhergehenden Ansprüche, **dadurch gekennzeichnet, dass** das Verschlusselement (6) zumindest einen vorstehenden Abschnitt (9) umfasst, der eng in die Regulierungsöffnung (5) eingesetzt werden kann.

5. Minderer (1) nach Anspruch 4, **dadurch gekennzeichnet, dass** der vorstehende Abschnitt (9) des Verschlusselements in der geschlossenen Position in die Regulierungsöffnung (5) eingesetzt und darin verriegelt wird.

6. Minderer (1) nach einem oder mehreren der Ansprüche 4 und 5, **dadurch gekennzeichnet, dass** der vorstehende Abschnitt (9) sich in der geschlossenen Position zumindest teilweise über die Öffnung (5) hinaus innerhalb des röhrenförmigen Körpers (2) erstreckt.

7. Minderer (1) nach einem oder mehreren der vorhergehenden Ansprüche, **dadurch gekennzeichnet, dass** er zumindest ein flexibles Element (10) mit einem ersten Ende, das mit dem Verschlusselement (6) verbunden ist, und einem zweiten Ende, das mit dem röhrenförmigen Körper (2) verbunden ist, umfasst.

8. Minderer (1) nach einem oder mehreren der vorhergehenden Ansprüche, **dadurch gekennzeichnet, dass** die Öffnungen (5) entlang der Längsachse des röhrenförmigen Körpers (2) ausgerichtet sind und im Wesentlichen voneinander beabstandet sind.

9. Minderer (1) nach einem oder mehreren der vorhergehenden Ansprüche, **dadurch gekennzeichnet, dass** zumindest eines des ersten Endes (3) und des zweiten Endes (4) zumindest einen Kupplungsabschnitt umfasst, der geeignet ist, die Verbindungen mit dem Aspirationsgerät und/oder mit dem Endelement zu erleichtern.

## Revendications

1. Dispositif de réduction de pression (1) pour systèmes d'aspiration chirurgicale, comprenant au moins un corps tubulaire sensiblement allongé (2) ayant au moins une première extrémité (3) pouvant être reliée à au moins une unité d'aspiration chirurgicale et au moins une seconde extrémité (4) pouvant être reliée à au moins un élément d'aspiration d'extrémité, ledit corps tubulaire (2) comprend au moins un trou (5) pour la régulation de la pression d'aspiration, et ledit dispositif de réduction (1) comprend au moins un élément de fermeture (6) mobile entre une position fermée dudit trou de régulation (5) et une position ouverte dudit trou de régulation (5), **caractérisé par le fait qu'**il comprend une pluralité de trous de régulation (5) et une pluralité d'éléments de fermeture (6) respectifs.

2. Dispositif de réduction (1) selon la revendication 1, **caractérisé par le fait que** ledit élément de fermeture (6) comprend au moins une portion de préhension (7).

3. Dispositif de réduction (1) selon la revendication 2, **caractérisé par le fait que** ladite portion de préhension (7) comprend une mise en forme de surface (8) apte à faciliter la préhension.

4. Dispositif de réduction (1) selon une ou plusieurs des revendications précédentes, **caractérisé par le fait que** ledit élément de fermeture (6) comprend au moins une portion en saillie (9) insérable de manière ajustée dans ledit trou de régulation (5).

5. Dispositif de réduction (1) selon la revendication 4, **caractérisé par le fait que**, dans ladite position fermée, ladite portion en saillie (9) de l'élément de fermeture est insérée et verrouillée à l'intérieur dudit trou de régulation (5).

6. Dispositif de réduction (1) selon une ou plusieurs des revendications 4 et 5, **caractérisé par le fait que** ladite portion en saillie (9), dans ladite position fermée, s'étend au moins en partie au-delà dudit trou (5), à l'intérieur dudit corps tubulaire (2).

7. Dispositif de réduction (1) selon une ou plusieurs des revendications précédentes, **caractérisé par le fait qu'**il comprend au moins un élément flexible (10) ayant une première extrémité associée audit élément de fermeture (6) et une seconde extrémité associée audit corps tubulaire (2).

8. Dispositif de réduction (1) selon une ou plusieurs des revendications précédentes, **caractérisé par le fait que** lesdits trous (5) sont alignés le long de l'axe longitudinal dudit corps tubulaire (2) et sont sensiblement espacés les uns des autres.

9. Dispositif de réduction (1) selon une ou plusieurs des revendications précédentes, **caractérisé par le fait qu'**au moins une de ladite première extrémité (3) et de ladite seconde extrémité (4) comprend au moins une portion de couplage capable de faciliter les liaisons avec ladite machine d'aspiration et/ou avec ledit élément d'extrémité.
